# EUROPEAN PATENT APPLICATION

(11) **EP 1 120 416 A1**
(43) Date of publication of application: **01.08.2001**
(21) Application number: 01200454.5
(22) Date of filing: 05.04.1994
(51) Int. Cl.: C07D 471/04, A61K 31/55

(54) **Pyrido[2.3-b][1,4]benzodiazepinones as M2 receptor ligand for the treatment of neurological disorders**

(30) Priority: 05.04.1993 US 42872
(62) Divisional of application: 94914039.6
(71) Applicant: PHARMACEUTICAL DISCOVERY CORPORATION, Elmsford, NY 10523 (US)
(72) Inventor: McCabe, R. Tyler, South Salem, New York 10590 (US); Wilson, Bryan R., Tuxedo, New York 10987 (US); Rhodes, Christopher A., Stamford, Connecticut 06902 (US)
(74) Representative: Dee, Ian Mark

(57) **Abstract**

Muscarinic M₂ receptor ligands are described which are suitable for the treatment of neurological disorders, and which may be administered with minimal side-effects. A method of synthesis of these compounds is also described.

## Description

### Background of the Invention

The present invention is in the field of neural receptors, and more particularly in the area of ligands to the muscarinic₂ receptor.

### Muscarinic Cholinergic Receptors

Acetylcholine triggers two classes of membrane receptors: nicotinic and muscarinic. The nicotinic receptor is a cation-specific channel in postsynaptic membranes, while the muscarinic receptor is a seven-helix transmembrane protein that triggers G proteins. Muscarinic cholinergic receptor blockade impairs memory performance, whereas enhancing muscarinic receptor activity improves memory function.

Molecular biological studies have indicated the existence of at least five subtypes of muscarinic cholinergic receptor, as reviewed by Bonner, T.I., Trends Neurosci, 12:145-151 (1989). The messenger RNA (mRNA) associated with the synthesis of these receptor subtypes has been localized using the technique of *in situ* hybridization, described by Buckley, N.J., *et al.,* J. Neurosci, 8:4646-4652 (1989). These muscarinic receptors are widespread throughout the central nervous system (CNS), except for the M₂ receptor, which appears to be relatively specialized and found in only a few unique regions, as reported by Liao, C.-F., *et al.*, J. Biol. Chem., 264:7328-7337 (1989). The existence of muscarinic cholinergic receptor subtypes holds the promise of developing new drugs which target only one receptor while not affecting others. The many side effects of cholinergic compounds developed to date, reviewed by Colleton, D., Neurosci, 19(1):1-28 (1986), make this approach a very desirable one. A more specific drug which acts at only one receptor subtype would be expected to be more focused on its effects.

### The Muscarinic₂ Receptor

The release of acetylcholine (ACh) appears to be modulated by presynaptic receptors of the muscarinic₂ (M₂) type. Over the past several years, evidence has accumulated that M₂ receptors are presynaptic in location at several sites on the CNS, as reviewed by Spencer, D.G., *et al*., Brain Res., 380:59-68 (1986), and these sites are reduced in Alzheimer's disease, as reported by Mash, D.C., *et al*., Science, 288:1115-1117 (1985), and Quirion, R., *et al*., TIPS, Dec. Suppl. 80-84 (1989). The presynaptic M₂ receptors on cholinergic neurons represent autoreceptors which regulate the release of ACh from the cholinergic neuron, as reported by Pittel, Z., *et al*., J. Neurochem., 55:665-672 (1990). Use of a specific M₂ ligand, which would block these sites, would be expected to increase the levels of ACh at the synapse, thereby increasing the effectiveness of ACh as a neurotransmitter at the synapse and in areas where its concentration has been reduced by loss of cholinergic neurons as seen in Alzheimer's Disease. Several relatively specific M₂ antagonists have been developed. However, these antagonists do not cross the blood-brain barrier.

### Neurological Disorders:

### Dementia

Dementia is a progressive decline in mental function, memory, and in acquired mental skills. About 11% of people in the United States over 65 years of age show mild to severe mental impairment, and from age 75 on the incidence of dementia is 2% higher with each year of life. Currently over one million people in the United States suffer serious dementia, and an estimated 120,000 of those die each year of causes related to severe dementia, as reported by Goldman, J. and Côté, L., Principles of Neural Science, 3rd Edition, ed. Kandel, *et al.*, 974-983 (Elsevier New York 1991).

About 70% of all cases of dementia are due to Alzheimer's disease, while a small percentage of cases are associated with other neurodegenerative diseases, such as Huntington's disease, or Parkinson's disease.

### Alzheimer's Disease

Alzheimer's Disease (AD) is characterized by cell loss in the cholinergic system, in the septohippocampal pathway, and in the projections from the nucleus basalis to the cerebral cortex, Colleton, D., Neurosci., 19(1):1-28 (1986), and Smith, G., Brain Res. Rev., 13:103-118 (1988). The involvement of cholinergic drugs in memory function has been well documented. For example, muscarinic cholinergic antagonists such as scopolamine impair memory function. Several cholinergic agonists have been developed which affect binding at muscarinic receptor sites. Cholinergic agonists and anticholinesterases have been successfully employed to improve memory in selected patients, as reported by Colleton, D., Neurosci., 19(1):1-28 (1986). These compounds, however, are short-acting and their side-effects soon become intolerable to the treated patients, as reported by Colleton, D., Neurosci., 19(1):1-28 (1986).

It is therefore an object of the present invention to provide compounds useful for the treatment of neurological disorders.

It is a further object of the present invention to provide a method of treatment of neurological disorders.

It is another object of the present invention to provide compounds for the treatment of dementia that cross the blood-brain barrier in an effective, therapeutic manner, and which may be administered either orally, intravenously, or parenterally.

It is another object of the present invention to provide compounds for the treatment of dementia which minimize the side effects associated with current methods of treatment for neurological disorders, and which are suitable for long-term treatment.

### Summary of the Invention

Muscarinic M₂ receptor ligands are described which are suitable for the treatment of neurological disorders, and which may be administered with minimal side-effects.

The compounds described herein have the following general structures: 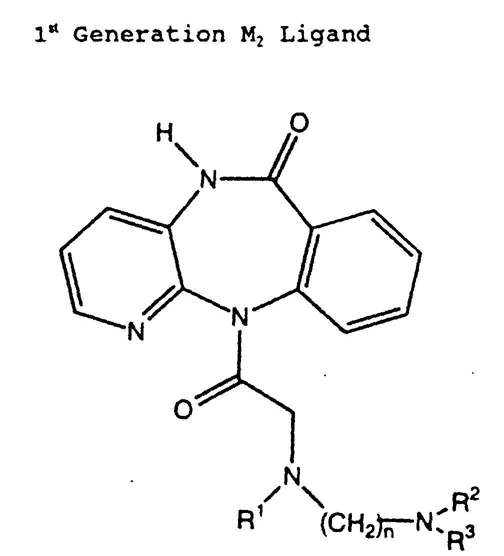 R¹, R², and R³ independently =
alkyl such as methyl, isopropyl, or t-butyl; cycloalkyl such as cyclopentyl, cyclohexylmethyl, or adamantyl; arylalkyl such as benzyl, napthylmethyl, or phenylpropyl; but are not limited to these alkyl, cycloalkyl, or arylalkyl groups, and are optionally substituted with the following groups: alkyl, aryl, alkoxy, aryloxy, amino, alkylthio, arylthio, halogen, nitro, alkyl or aryl carbonyl, alkyl or aryl sulfonyl, alkyl or aryl sulfamido, alkyl or aryl carbonamido, alkyl or arylurea;
wherein n is between 1 and 20. 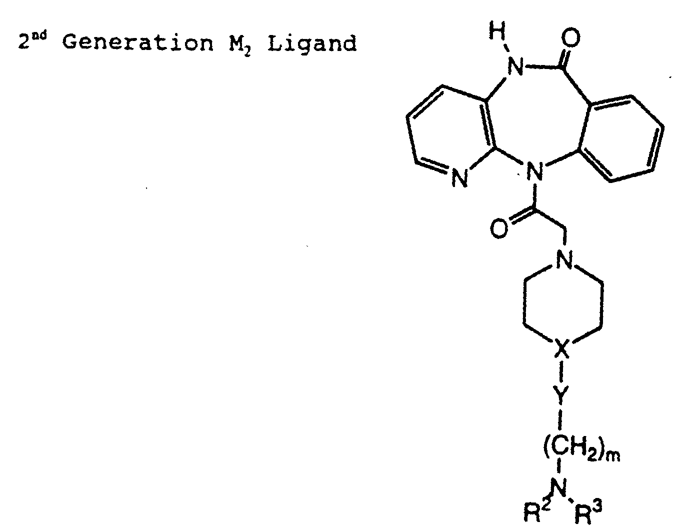
X = -CH, N;
Y = -CONR¹-, -NR¹CO-, -CH₂NR¹-, -NR¹CH₂-, -NCO(CH₂)ₙNR;
R¹ = alkyl such as methyl, isopropyl, or t-butyl; cycloalkyl such as cyclopentyl, cyclohexylmethyl, or adamantyl; arylalkyl such as benzyl, napthylmethyl, or phenylpropyl; alkyl- or arylcarbonyl, such as cyclohexane carbonyl or benzoyl; alkyl- or aryl sulfonyl, such as methane sulfonyl or napthalenosulfonyl; alkyl- or arylurea, such as cyclohexylurea or phenylurea; but are not limited to these alkyl or aryl groups, and are optionally substituted with the following groups: alkyl, aryl, alkoxy, aryloxy, amino, halogen, nitro, alkyl or aryl carbonyl, alkyl or aryl sulfonyl, alkyl or aryl sulfamido, alkyl or aryl carbonamido, alkyl or arylurea;
R², and R³ = alkyl such as methyl, isopropyl, or t-butyl; cycloalkyl such as cyclopentyl,
cyclohexylmethyl, or adamantyl; arylalkyl such as benzyl, napthylmethyl, or phenylpropyl; but are not limited to these alkyl, aryl cycloalkyl, or arylalkyl groups, and are optionally substituted with the following groups: alkyl, aryl, alkoxy, aryloxy, amino, halogen, nitro, alkylthio or arylthio, alkyl or aryl carbonyl, alkyl or aryl sulfonyl, alkyl or aryl sulfamido, alkyl or aryl carbonamido, alkyl or arylurea;
wherein m and n is between 1 and 20.

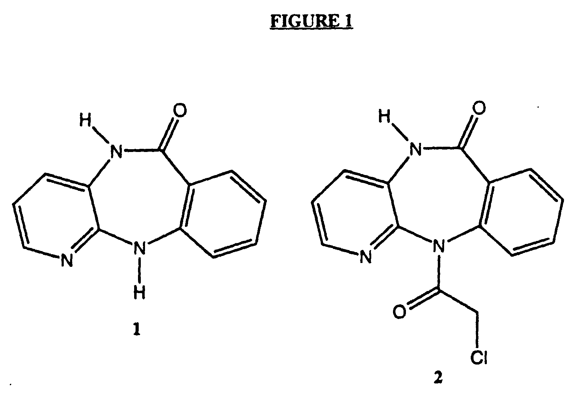

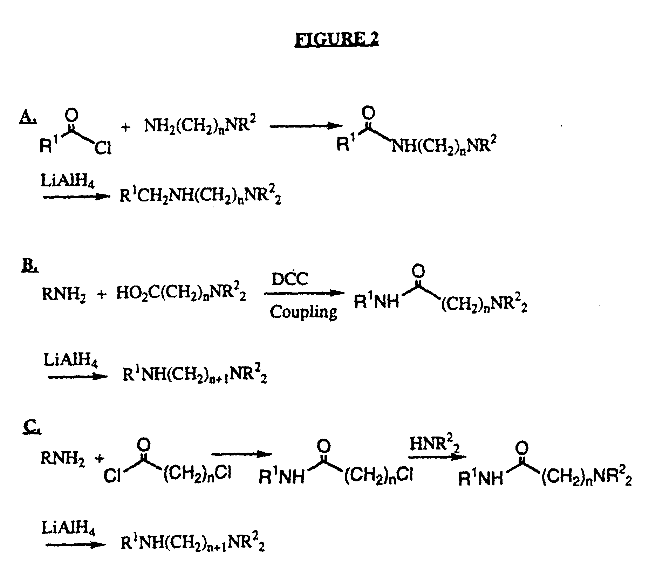

The first-generation M₂ ligands are produced by reaction of compound 2 (Figure 1) with an excess of a secondary amine. The secondary amine may be produced by three routes: (1) reaction of acid chloride with N,N-dialkyl diamino alkanes, (2) reaction of a primary amine and an activated N,N-dialkyl alkanoic acid, and (3) reaction of a primary amine with a chloroalkanoyl chloride, yielding an alkylchloride, which is then reacted with a secondary amine.

The second generation M₂ ligands are produced by reaction of compound 2 (figure 1) with secondary cycloalkylamines. The secondary cycloalkylamines may be produced by reaction of N-protected isonipecotic acid, N-benzylpiperazine, or N-benzyl-4-piperidone with the secondary amine produced in the first generation compounds. The products of these reactions are N-deprotected and reacted with compound 2 (Figure 1).

The compounds can be administered orally, intraperitoneally, intravenously, intramuscularly, or topically in a therapeutically effective dosage, i.e., producing enhancement of cognitive function with a therapeutic index of 2 or greater, most preferably orally once daily in a dosage of 10 mg compound/kg of body weight in order to enhance cognition in a subject in need of such enhancement. No toxic side effects have been observed using therapeutically effective doses. Examples demonstrate effectiveness of several compounds in standard pharmacological *in vivo* tests.

### Brief Description of the Drawings

Figure 1 are tricyclic aromatic precursors to muscarinic₂ antagonist compounds.

Figures 2A, 2B, and 2C are schematics of three routes to the synthesis of a secondary amines used in the synthesis of muscarinic₂ antagonist compounds. Figure 2A uses acid chloride plus diamine, followed by reduction. Figure 2B uses amine plus dialkylamino acid, followed by reduction. Figure 2C uses amine plus chloroalkylacid chloride, then secondary amine, followed by reduction.

Figure 3 is a schematic of the syntheses of second generation muscarinic₂ compounds.

Figure 4 depicts one embodiment of the synthesis of 11-[[[2-(diethylamino)ethyl] cyclohexylmethylamino]-acetyl]-5,11-dihydro-6H-pyrido [2,3-b] [1,4]-benzodiazepin-6-one (referred to herein as "PDC008.004").

Figure 5 is a graph of the inhibition of [³H]AF-DX-384 binding to M₂ receptors in the rat brain by PDC008.004, determined as % specific [³H]AFDX 384 binding versus log [PDC008.004] in nM.

Figure 6 is a graph of the dose-response of PDC008.004 on evoked hippocampal Acetylcholine release, acetylcholine release as percent of control versus concentration (M) of PDC008.004 (-open squares-) and AF-DX 116 (-closed squares-).

Figure 7 is a graph of the effect of various doses of PDC008.004 on heart rate in male, Sprague-Dawley rats which received cumulative doses of PDC008.004 (0.1 to 31.1 mg/kg) (stippled bars), where heart rate was assessed by EKG approximately 15 minutes after each dose. Control animals received an equal volume of vehicle (Emulphor:saline:DMSO, 18:72:10%) (closed bar) or (-)norepinephrine bitartrate (1 mg/rat) (open bar).

Figure 8 is a graph of the average movement pattern changes in PDC 008.004 treated mice and in untreated (control) mice in a watermaze. Movement type: 1, wall hug; 2, wall/cross; 3, cross; 4, cross/goal; and 5, goal.

Figure 9 is a graph of target latencies (seconds) after the fourth trial in PDC 008.004 treated mice (stippled bar) and untreated (control) (open bar) mice in a watermaze.

Figure 10 is a graph of time to target (seconds) over time (days) for control (open circles) and PDC008.004 treated animals (closed circles).

### Detailed Description of the Invention

Muscarinic (M)₂ receptor ligands are described which are suitable for the treatment of neurological disorders, and which may be administered with minimal side-effects. A method of administration of these compounds to treat dementia is also described, as well as a method of synthesis of these compounds.

The compounds are suitable for the treatment of dementia associated with, but not limited to, the following conditions: Alzheimer's disease, Parkinson's disease, Huntington's disease, senile dementia, alcoholism, meningitis, neonatal hypoxia, stroke, or global cerebral ischemia.

### Synthesis of M₂ Receptor Ligands

As used herein, the following definitions apply:
The term "alkyl," unless otherwise defined, refers to a C₁ to C₂₀ straight or branched alkyl group, and specifically includes methyl, isopropyl, and t-butyl.
The term "cycloalkyl," unless otherwise defined, refers to a cyclic alkyl group of from 3 to 10 carbon atoms;
The term "alkoxy," unless otherwise defined, refers to a moiety of the structure o-alkyl;
The term "aryl," unless otherwise defined, refers to a phenyl or benzyl, or substituted phenyl, or substituted benzyl, wherein the substituent is (A) alkyl, such as methyl, isopropyl, or t-butyl; (B) cycloalkyl, such as cyclopentyl, cyclohexylmethyl, or adamantyl; arylalkyl, such as benzyl, napthylmethyl, or phenylpropyl; alkyl- or arylcarbonyl, such as cyclohexane carbonyl or benzoyl; alkyl- or aryl sulfonyl, such as methane sulfonyl or napthalenosulfonyl; alkyl- or arylurea, such as cyclohexylurea or phenylurea; and (C) optionally substituted with the following groups: alkyl, aryl, alkoxy, aryloxy, amino, halogen, nitro, alkyl or aryl carbonyl, alkyl or aryl sulfonyl, alkyl or aryl sulfamido, alkyl or aryl carbonamido, alkyl or arylurea.

The compounds are predicted to selectively bind to the M₂ receptor with an affinity in the nanomolar range, and have low toxicity (LD₅₀ less than 37.5 mg/kg, intraperitoneally, and less than 45 mg/kg, orally). These compounds have a molecular weight range of between 300 and 1500 grams/mol. As defined herein, the compounds that are useful for treatment of a patient are those having a therapeutic index of 2 or greater, wherein the therapeutic index is the lethal dosage (LD)₅₀ divided by the effective dosage (ED)₅₀.

The compounds are also predicted to be acid-stable, in order to make possible oral administration.

The modified compounds described herein have the following general structures: 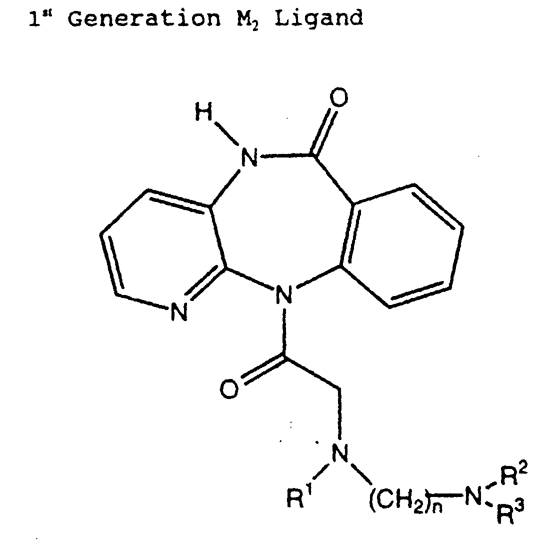 R¹, R², and R³ independently =
alkyl such as methyl, isopropyl, or t-butyl; cycloalkyl such as cyclopentyl, cyclohexylmethyl, or adamantyl; arylalkyl such as benzyl, napthylmethyl, or phenylpropyl; but are not limited to these alkyl, cycloalkyl, or arylalkyl groups, and are optionally substituted with the following groups: alkyl, aryl, alkoxy, aryloxy, amino, alkylthio, arylthio, halogen, nitro, alkyl or aryl carbonyl, alkyl or aryl sulfonyl, alkyl or aryl sulfamido, alkyl or aryl carbonamido, alkyl or arylurea;
wherein n is between 1 and 20. 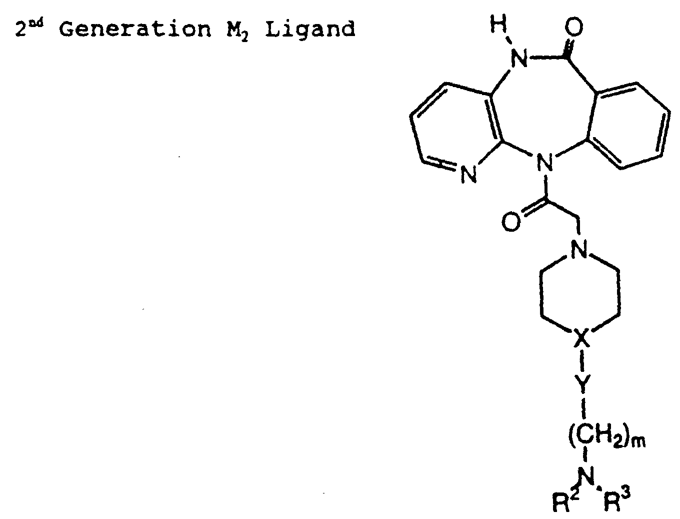
X = -CH, N;
Y = -CONR¹-, -NR¹CO-, -CH₂NR¹-, -NR¹CH₂-, -NCO(CH₂)ₙNR;
R¹ = alkyl such as methyl, isopropyl, or t-butyl; cycloalkyl such as cyclopentyl, cyclohexylmethyl, or adamantyl; arylalkyl such as benzyl, napthylmethyl, or phenylpropyl; alkyl- or arylcarbonyl, such as cyclohexane carbonyl or benzoyl; alkyl- or aryl sulfonyl, such as methane sulfonyl or napthalenosulfonyl; alkyl- or arylurea, such as cyclohexylurea or phenylurea; but are not limited to these alkyl or aryl groups, and are optionally substituted with the following groups: alkyl, aryl, alkoxy, aryloxy, amino, halogen, nitro, alkyl or aryl carbonyl, alkyl or aryl sulfonyl, alkyl or aryl sulfamido, alkyl or aryl carbonamido, alkyl or arylurea;
R², and R³ = alkyl such as methyl, isopropyl, or t-butyl; cycloalkyl such as cyclopentyl, cyclohexylmethyl, or adamantyl; arylalkyl such as benzyl, napthylmethyl, or phenylpropyl; but are not limited to these alkyl, aryl cycloalkyl, or arylalkyl groups, and are optionally substituted with the following groups: alkyl, aryl, alkoxy, aryloxy, amino, halogen, nitro, alkylthio or arylthio, alkyl or aryl carbonyl, alkyl or aryl sulfonyl, alkyl or aryl sulfamido, alkyl or aryl carbonamido, alkyl or arylurea;
   wherein m and n is between 1 and 20.

### Synthesis of First General M₂ Ligands

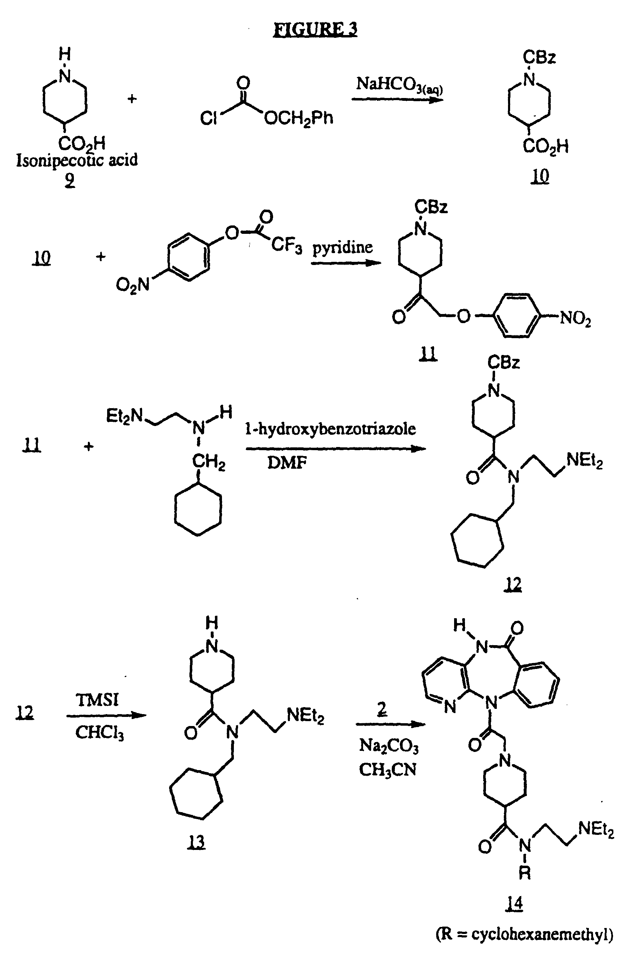

The first generation M₂ ligands are produced by reaction of compound 2 (Figure 1) with an excess of a secondary amine. The secondary amine may be produced by three routes: (1) Reaction of acid chloride with N,N-dialkyl diamino alkanes, (2) reaction of a primary amine and an activated N,N-dialkyl alkanoic acid, and (3) reaction of a primary amine with a chloroalkanoyl chloride, yielding an alkylchloride, which is then reacted with a secondary amine. (Figure 2).

### Synthesis of Second Generation M₂ Ligands

The second generation M₂ ligands are produced by reaction of compound 2 (Figure 1) with secondary cycloalkylamines. The secondary cycloalkylamines may be produced by reaction of N-protected isonipecotic acid, N-benzylpiperazine, or N-benzyl-4-piperidone with the secondary amines produced in the first generation compounds. The products of these reactions are N-deprotected and reacted with compound 2 (Figure 1).

### Pharmaceutical Compositions

The compounds are administered topically, locally, or systemically, depending on the application. When administered systemically, the compound is preferably administered orally or intravenously, although it can be administered intraperitoneally, intramuscularly, or topically in an appropriate pharmaceutical carrier such as normal saline, or in tablet form. For topical application, the compound is preferably administered in an ointment or cream base, or by means of a transdermal patch. The compound can also be administered by controlled delivery devices, such as biodegradable polymers, or by inhalation, insufflation, or nasal spray. Suitable carriers are known to those skilled in the pharmaceutical area.

The compounds are administered orally, intraperitoneally, intramuscularly, subcutaneously, or topically in a therapeutically effective dosage to enhance cognition or otherwise alleviate the symptoms of the neurological disorder. In the most preferred embodiment, the compounds are administered orally once daily in a dosage range equivalent to 10 mg/kg. For mice, the LD₅₀ for oral administration is 45 mg/kg, and the LD₅₀ for intraperitoneal administration is 37.5 mg/kg. Only those compounds having a therapeutic index of 2 or greater are administered to a patient.

The concentration of active compound in the drug composition will depend on absorption, inactivation, and excretion rates of the drug as well as other factors known to those skilled in the art. It is to be noted that dosage values will also vary with the condition of the subject being treated. It is to be further understood that for any particular subject, specific dosage regimens should be adjusted over time according to the individual need and the professional judgment of the person administering or supervising the administration of the compositions, and that the concentration ranges set forth herein are exemplary only and are not intended to limit the scope or practice of the claimed composition.

For oral delivery, the compounds may be enclosed in capsules, compressed into tablets, microencapsulated, entrapped in liposomes, in solution or suspension, alone or in combination with a substrate-immobilizing material such as starch or poorly absorbable salts such as immodium. Pharmaceutically compatible binding agents can be included as part of the composition. The tablets or capsules may contain, for example, any of the following ingredients, or compounds of a similar nature: a binder such as microcrystalline cellulose, gum tragacanth or gelatin; an excipient such as starch or lactose, a disintegrating agent such as alginic acid, Primogel®, or corn starch; a lubricant such as magnesium stearate or Sterotes; a glidant such as colloidal silicon dioxide; a sweetening agent such as sucrose or saccharin; or a flavoring agent such as peppermint, methyl salicylate, or orange flavoring. When the dosage unit form is a capsule, it can contain, in addition to material of the above type, a liquid carrier. In addition, dosage unit forms can contain various other materials which modify the physical form of the dosage unit, for example, coatings of sugar, shellac, or other enteric agents.

The present invention will be further understood with reference to the following nonlimiting examples.

### Example 1: synthesis of M₂ receptor ligands.

The compounds described herein have a tricyclic aromatic ring system. The tricyclic aromatic ring system is attached to an amine-containing side-chain via an acetyl group. The tricyclic aromatic 5,11-dihydro-6H-pyrido [2,3-b] [1,4]-benzodiazepin-6-one, **1** and its chloroacetyl derivative 5,11-dihydro-11-(chloroacetyl)-6H-pyridol [2,3-b] [1,4]-benzodiazep in-6-one, **2** were prepared according to standard procedures and had physical and spectroscopic properties identical to the values reported by Engel, W.W., *et al*., J. Med. Chem. 32:1718-1724 (1989); U.S. Patent No. 3,406,168, 1968. The chloroacetyl derivative **2**, was used in all of the syntheses. See Figure 1 for structures.

### a. First Generation M₂ Ligands

The first M₂ ligands produced have the general structure shown in the formula described above. They were produced in 65-85% yield by reaction of the acetyl chloride **2** with an excess of a secondary amine. The secondary amines can be produced via three routes, all proceeding through an amide which is reduced in the final step to yield the secondary amine, as shown by Figure 2.

The first route, shown in Figure 2A, involves the reaction of acid chlorides with N,N-dialkyl diamino alkanes. The second route, shown in Figure 2B, involves the reaction of a primary amine and an activated N,N-dialkyl alkanoic acid. N,N-Dicyclohexylcarbodiimide or some other agent known to those skilled in the art could be used to activate the acid to nucleophilic attach by the amine. In the third route, shown in Figure 2C, a primary amine is reacted with a chloroalkanoyl chloride giving an alkylchloride which is then reacted with a secondary amine such as diethylamine to produce the desired amide.

All reagents used in the secondary amine synthesis are readily available from Aldrich Chemical Co., Inc., Milwaukee, WI.

### Synthesis

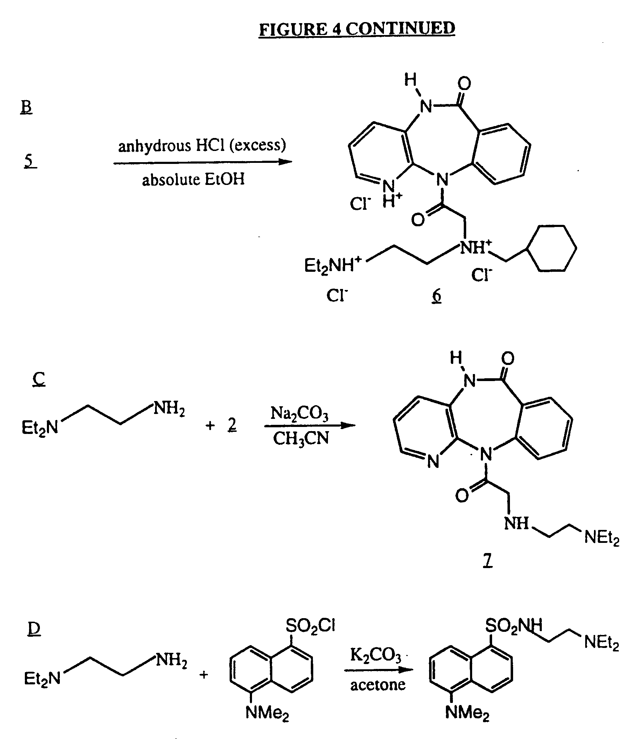

The synthesis of PDC 008.004 and related compounds is shown in Figure 4.

N-[2-(Diethylamino)ethyl]-cyclohexane carboxamide; **3**: Cyclohexane carbonyl chloride (55.9 mmol) was dissolved with stirring in 650 mL acetone in a one-liter three-necked round-bottomed flask. Anhydrous potassium carbonate (55.0 mmol) was added to the flask in one portion. A condenser, 125 mL addition funnel and stopper was placed in each of the three necks. N,N-diethyl ethylenediamine (43.0 mmol) was dissolved in 100 mL acetone and transferred to the addition funnel. While the reaction mixture was stirred at 25°C, the amine/acetone solution was added over a 1.6 hour period. After 19 hours reaction at 25°C, all of the cyclohexane carbonylchloride was consumed producing a new spot by thin-layer chromatography at Rp 0.25 (silica gel GF, 250 µm, 1:9:90 ammonium hydroxide: methanol: chloroform, iodine visualization). Solids were filtered and washed with acetone and the acetone was evaporated on a rotary evaporator. The residue was dissolved in 200 mL chloroform and extracted with three 100 mL portions of 10% aqueous sodium bicarbonate. The combined bicarbonate washes were back-extracted with 300 mL chloroform and the combined chloroform layers were washed with 100 mL distilled water. The chloroform was evaporated and residual solvent was removed under vacuum with gentle heating, yielding off-white crystals. Recrystallization from hexanes and ethyl acetate gave white crystals which had a melting point of 105-107°C (30.9 mmol, 72% yield). Spectroscopic properties were consistent with the proposed structure.

N-[2-(diethylamino)ethyl] cyclohexanemethyl amine, **4**: Compound **3** (11.2 mmol) was dissolved in 150 mL diethyl ether in a 250 mL flask which was purged with argon. Lithium aluminum hydride (16.8 mmol, 1.0 M in diethyl ether) was added in one portion via a gas-tight syringe. After one hour, compound **3** (R_{f} 0.5) is completely consumed, producing one new spot at R_{f} 0.4 (silica gel GF, 250 µm, 1:9:90 ammonium hydroxide: methanol: chloroform). To quench any remaining hydride, 0.65 mL water was added with stirring, then 0.65 mL 15% aqueous sodium hydroxide, then 1.95 mL water. The white precipitate was filtered and washed with 100 mL diethyl ether. The ether was dried over sodium sulfate and evaporated with only gentle heating on a rotary evaporator. A clear colorless liquid was obtained (9.05 mmol, 81% yield) which had spectroscopic properties consistent with the proposed structure.

PDC 008.004, **5**: To a 500 mL round-bottomed flask containing a stirbar was added 4.41 mmol **2,** 7.06 mmol **4,** 7.06 mmol sodium carbonate and 170 mL acetonitrile. Not all of **2** was soluble at 25°C. Upon heating to reflux (90°C), all solids dissolve. After 22 hours, thin layer chromatography (silica gel GF, 250 µm, 1:9:90 ammonium hydroxide: methanol: chloroform) shows that **2** (R_{f} 0.5, ultraviolet and iodine active) is completely consumed, producing a new spot at Rf 0.2 (ultraviolet and iodine active). Some unreacted **4** (R_{f} 0.4, iodine active) is still present. As the solution cools to room temperature, white solids precipitate from acetonitrile. The sodium carbonate is filtered and washed with acetonitrile. The solvent is evaporated and recrystallized from acetonitrile to obtain white needle-like crystals with a melting point of 208-209°C (3.77 mmol, 86% yield). This material had spectroscopic properties consistent with the proposed structure. The extinction coefficient at 285 nm in 1-octanol is 8199 ± 616 M⁻¹cm⁻¹. The partition coefficient (log P) between 1-octanol and 0.1 M phosphate is 0.78 ± 0.18.

11-[[[2-(Diethylamino) ethyl] cyclohexylmethylamino]-acetyl]-5,11-dihydro-6H-pyrido [2,3-b] [1,4]-benzodiazepin-6-one hydrochloride salt, **6**: Compound **5** (1.29 mmol) was converted to its hydrochloride salt by dissolving it in absolute ethanol saturated with anhydrous hydrogen chloride until the pH of the solution was less than 2 (approximately 10 mL). The salt was crystallized by adding 100 mL anhydrous diethyl ether to the boiling ethanolic solution. The white powdery solids which formed were filtered and washed with two 50 mL portions of ether. This procedure was repeated and a fine white powder was obtained which decomposed at 190°C before it melted (1.22 mmol, 94% yield). The white solid had physical and spectroscopic properties consistent with the proposed structure. The extinction coefficient at 281 nm in 0.1 M phosphate is 9060 M⁻¹cm⁻¹. The partition coefficient (log P) between 0.1 M phosphate and 1-octanol is 0.16.

### b. Synthesis of Related Compounds

11-[[[2-(Diethylamino) ethyl]amino]-acetyl]-5,11-dihydro-6H-pyrido [2,3-b] [1,4]-benzodiazepin-6-one, **7:** N,N-diethylethylenediamine (10.7 mmol) and **2** (3.52 mmol) were dissolved in 200 mL acetonitrile containing sodium carbonate (5.22 mmol) and a stirbar. The mixture was refluxed for 18 hours at 95°C. Thin-layer chromatography (silica gel GF, 250 µm, 1:9:90 ammonium hydroxide: methanol: chloroform) showed almost complete consumption of 2 (R_{f} 0.55, UV and iodine active), the presence of some N,N-diethyl ethylenediamine (R_{f} 0.1, iodine active) and a new major spot at R_{f} 0.45 (UV and iodine active). Solids were filtered from the reaction mixture and washed with acetonitrile. The solvent was evaporated and the residue was dissolved in ethyl acetate and 10% aqueous sodium bicarbonate. 125 mL ethyl acetate solution was washed with three 50 mL portions 10% aqueous bicarbonate. Bicarbonate was back extracted with ethylacetate (75 mL), organics combined and washed with 100 mL distilled water. The ethyl acetate solution was dried over sodium sulfate, filtered and the solvent evaporated. The residue obtained was recrystallized from ethanol and water to yield an off-white solid with a melting point of 96-97°C (0.206 mmol, 6% yield). The physical and spectroscopic properties were consistent with the proposed structure.

N-[2-(diethylamino)ethyl]-5-dimethylamino-1-naphthalene sulfonamide, **8**: 5-Dimethylamino-1-napthalenesulfonylchloride, DANSYL chloride (0.56 mmol) was dissolved in 5 mL acetone with stirring at 25°C. Anhydrous potassium carbonate (0.56 mmol) was added in one portion and N,N-diethyl ethylenediamine (0.43 mmol) in 3 mL acetone was added over a five minute period from an additional funnel. After 18 hours at 26°C, thin-layer chromatography (silica gel GF, 250 µm, 1:9:90 ammonium hydroxide: methanol: chloroform) showed some N,N-diethyl ethylenediamine (R_{f} 0.25, iodine active), some DANSYL chloride (R_{f} 0.0, UV active) and a major new fluorescent yellow spot at R_{f} 0.65 (iodine and UV active). Solids were filtered and washed with acetone and the acetone was evaporated. The fluorescent spot at R_{f} 0.65 was isolated by preparative thin layer chromatography (silica gel G, 1000 µm, 1:9:90 ammonium hydroxide: methanol: chloroform). The largest band at R_{f} 0.45-0.65 was removed from the silica with 250 mL ethylacetate. The ethylacetate was washed with 100 mL water and dried over sodium sulfate. Evaporation of the solvent gave a yellow-green film (0.210 mmol, 49% yield) which had a proton NMR spectrum consistent with the proposed structure.

### c. Second Generation M₂ Ligands

The second generation M₂ ligands have the general structure described above. Like the first generation ligands, they have a tricyclic aromatic ring system linked via an acetyl group to the amine-containing sidechain. However, a small aliphatic ring has been incorporated in the sidechain in between the terminal amine and the aromatic **1**. The amide and amine functionality in the linker were chosen to allow the facile synthesis of a wide variety of linkers of varying chain length and physical and chemical properties. The nature of the terminal amine can also be varied, if desired.

The synthesis of the sidechains can be effected in a few steps from the readily available starting materials shown in Table 1. Aldrich Chemical Co., Inc., Milwaukee, WI. These precursors are shown only to demonstrate the wide variety of options available in the synthesis and they should not be taken as a complete list. Methods for coupling the various constituents of the sidechain together are known to those skilled in the art.

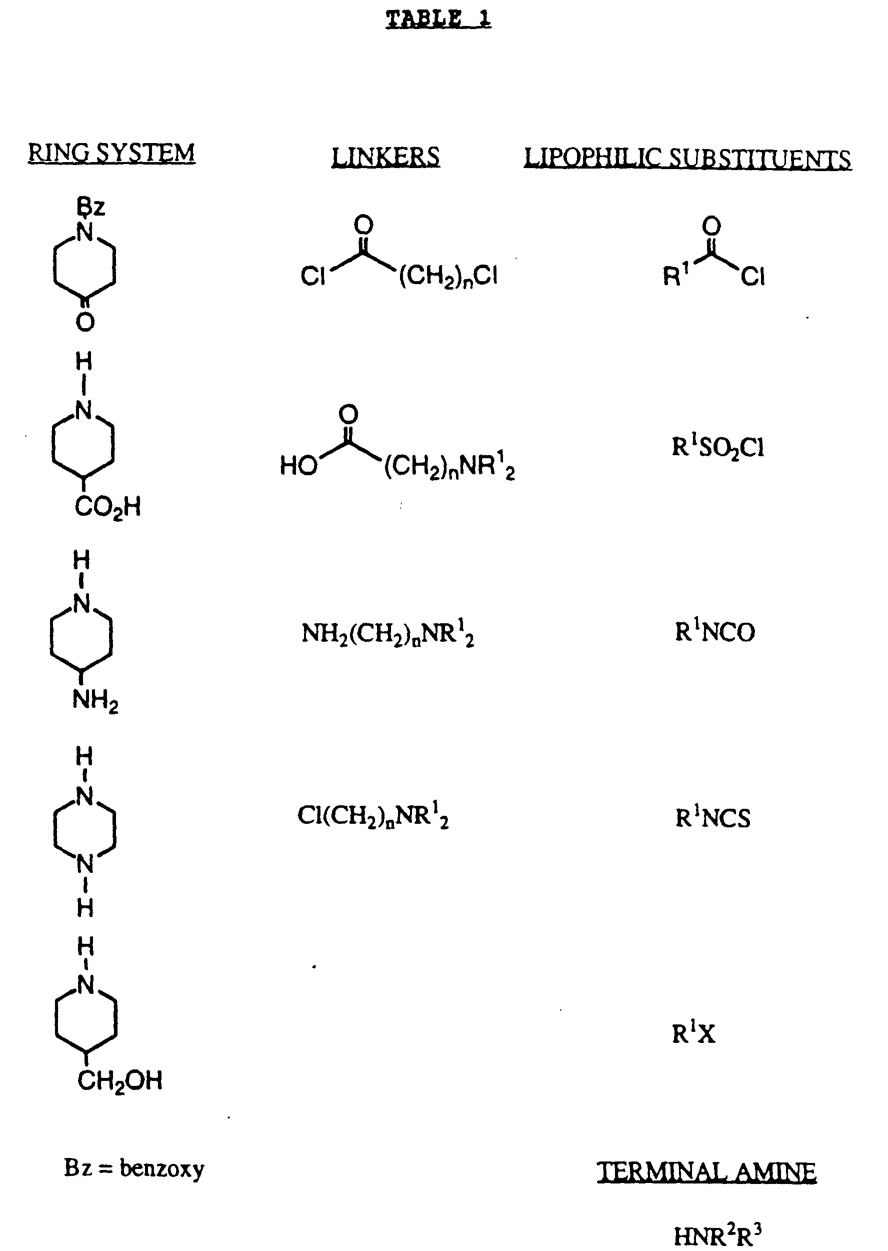

The ring system could come from the substituted piperidines or piperazine in the protected or unprotected forms. The linker is available, for example, from varying lengths of the diethyl amino alkanoic acids, the N,N-dialkyl diamines or the N,N-dialkyl alkylchlorides. In the aforementioned cases, these also incorporate the terminal amine and they are available in limited choices for R¹ such as ethyl, methyl, etc. For more flexibility, the chloroalkyl acid chlorides provide a means of varying the chain length and adding the desired secondary amine in another step. The wide range of secondary amines available makes this an attractive route. There are a large variety of alkyl and aryl groups readily available as the acid chloride, sulfonyl chloride, alkyl halide, isocyanate and isothiocyanate which are easily attached and could alter the properties of the ligands. Desirable properties which could be achieved through attachment of such active groups are listed as examples, but are not limited to the following: the ability to fluoresce, the presence of radioactive isotopes, changes in polarity, active groups for irreversible ligand binding (photoaffinity labelling), and fluorine-containing substituents for NMR imaging.

### Synthesis

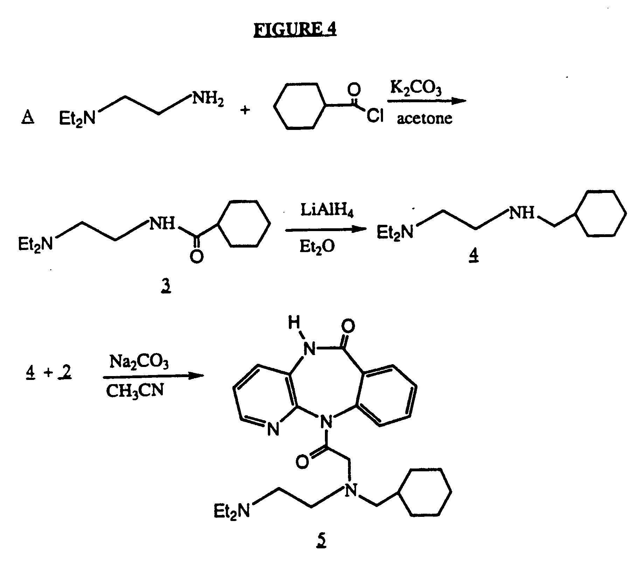

Figure 3 shows the synthesis of the second generation M₂ ligands starting from isonipecotic acid, **9**. Aldrich Chemical Co., Inc., Milwaukee, WI. The amino function is protected, the acid is activated to nucleophilic attach, reacted with the secondary amines produced for the first generation compounds (**4**, for example), deprotected and reacted with the acetyl chloride derivative of the tricyclic aromatic, **2** giving the desired compounds.

1-Benzyloxycarbonyl-4-piperidinecarboxylic acid, **10:** Compound **10** was prepared from isonipecotic acid, **9** using a procedure in Chemistry of the Amino Acids, Vol. 2, John Wiley and Sons, Inc., pp. 891-895 (New York 1961). Isonipecotic acid (7.74 mmol) was dissolved in 16 mL 10% aqueous sodium bicarbonate (19.3 mmol) with stirring. Benzylchloroformate (8.52 mmol) was added in five portions over a thirty minute period. The reaction mixture was stirred vigorously to effectively mix the benzylchloroformate with the aqueous solution. The mixture was stirred 18 hours at 25°C, at which time 10 mL water was added in an attempt to dissolve the cloudy white solids.' The aqueous solution was washed with 50 mL diethyl ether, neutralized to pH 3 with 5 N hydrochloric acid, and washed with two 30 mL portions of ethyl acetate. The ethyl acetate was dried over sodium sulfate, filtered and evaporated, yielding a clear, colorless, viscous oil (7.6 mmol, 98% yield). Thin layer chromatography (silica gel GF, 250 µm, 4:1:1 1-butanol: acetic acid: water), showed no starting material present (R_{f} 0.25, UV active), and only one spot at R_{f} 0.85 which is both UV and iodine active. The physical and spectroscopic properties of this oil are consistent with the structure proposed.

4-Nitrophenyl-1-benzyloxycarbonyl-4-piperidinecarboxylate, **11:** Compound **11** was produced from **10** using a procedure involving 4-nitrophenyl trifluoroacetate described by Sakahibara, S. and Innhai, N., Bull, Chim, Soc. Jpn., 37:1231-1232 (1964). Compound **10** (5.7 mmol) was dissolved in 4 mL pyridine with stirring. As soon as 4-nitrophenyl trifluoroacetate (5.7 mmol) was added, a yellow color developed. The reaction mixture was stirred for 20 hours at 25°C at which time thin layer chromatography (silica gel GF, 250 µm, 1:9:90 ammonium hydroxide: methanol: water) showed starting material **10** was almost completely consumed (R_{f} 0.1, UV and iodine active) producing a new major spot at R_{f} 0.9 which was both UV and iodine active. The reaction mixture was dissolved in 150 mL diethyl ether and washed with two 150 mL portions of 10% aqueous copper sulfate to remove pyridine, eight 100 mL portions of 10% aqueous sodium bicarbonate to remove 4-nitrophenol and two 100 mL portions of water. The diethyl ether was evaporated, leaving a clear, colorless oil which formed an off-white solid upon cooling in a -20°C freezer. This sample was recrystallized from hexanes and ethyl acetate. The physical and spectroscopic properties were consistent with the structure assigned.

N-[2-(Diethylamino)ethyl]-N-cyclohexanemethyl 1-benzyloxycarbonyl-4-piperidinecarboxamide, **12:** Compound **12** was prepared by the reaction of **4** with the activated ester **11** in the presence of 1-hydroxybenzotriazole as described by Glass, J.G. and Pelzig, M. Int. J. Peptide Protein Res., 12:75-80 (1978). To a solution of compound **4** (1 mmol) and 1-hydroxybenzotriazone (1 mmol) in 1 mL dimethylformamide was added the nitrophenyl ester, **11** (1 mmol). After 1 hour at 25°C, the reaction was diluted with 10 mL diethyl ether. A precipitate formed which was washed with diethyl ether, cold water and dissolved in hot water. On standing and cooling to room temperature an off-white solid formed. Physical and spectroscopic properties were consistent with the proposed structure.

N-[2-(Diethylamino)ethyl]-N-cyclohexame methyl 4-piperidine carboxamide, **13**: Deprotection of the benzyoxylcarbonyl protected piperidine was effected using trimethylsilyl iodine, TMSI, using the method of Lott, R.S., *et al*., J.C.S. Chem. Commun., 495-496 (1979). To a solution of **12** (0.1 mmol) in 1 mL chloroform was added TMSI (0.12 mmol) via a dry gas-tight syringe. The solution was heated to 50°C for 10 minutes at which time methanol (0.4 mmol) was added via a syringe to quench the reaction. Once the reaction mixture cooled to room temperature, the solvent was evaporated and the residue recrystallized from ethyl acetate and hexanes to yield an off-white solid. The physical and spectroscopic properties were consistent with the proposed structure.

11-[[N-(Diethylamino)ethyl]-N-cyclohexanemethyl 1-piperidine-4-carboxamido]-acetyl]-5,11-dihydro-6H-pyrido[2,3-b] [1,4]-benzodiazepin-6-one, **14:** To a solution of **13** (1.6 mmol) in 40 mL acetonitrile was added **2** (1.0 mmol) and sodium carbonate (1.6 mmol). The solution was heated to reflux for 18 hours. The solution was cooled to room temperature, the solids filtered and washed with acetonitrile and the solvent evaporated. The off-white solids obtained were recrystallized from acetonitrile. The white flaky solid obtained had physical and spectroscopic properties consistent with the proposed structure.

The following compounds were synthesized by similar methods to those described above.

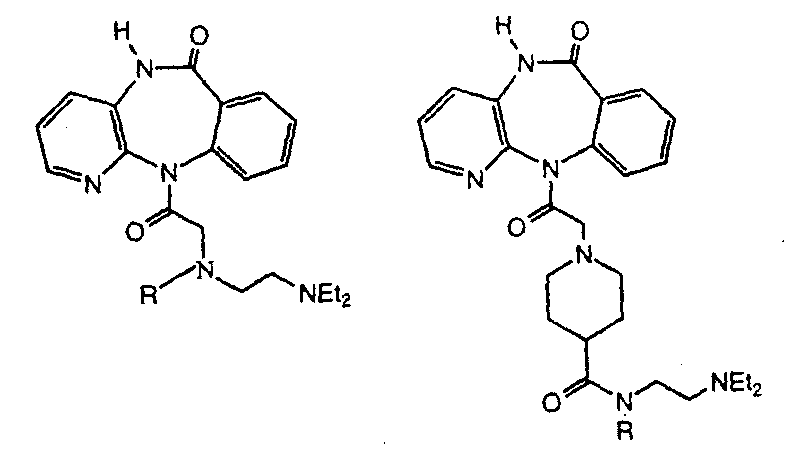

R = H

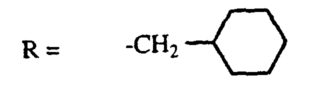

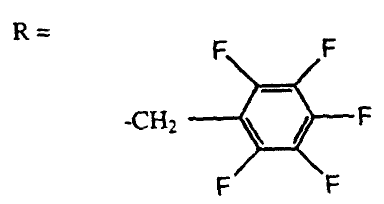

R = -CH₂(CH₂)₁₃CH₃

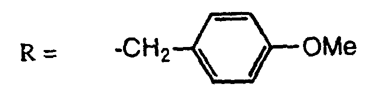

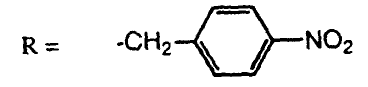

### Example 2: Physical and Chemical Characterization of multiple batches of 11-[[[2-(diethylamino)ethyl] cyclohexylmethylamino]-acetyl]-5,11-dihydro-6H-pyrido [2,3-b] [1,4]-benzodiazepin-6-one (PDC008.004).

To determine purity in scale-up of synthetic batches, thin layer chromatography and melting point are used for the first determination. Proton NMR and HPLC are then used to confirm structure and purity.
1. Nuclear Magnetic Resonance Spectrum of PDC008.004, batch PDC020.031 (¹H NMR, CDCl₃, ppm): 8.35 (1H), 7.95 (d,1H), 7.60 (m,4H), 7.45 (t,1H), 7.3 (dd,1H), 3.5 (m,2H), 2.60 (t,2H), 2.50 (q,4H), 2.40 (m,2H), 2.30 (d,2H), 1.60 (dd,4H), 1.25 (m,1H), 1.10 (m,4H), 0.95 (t,6H) 0.70 (m,2H). The spectra from batches PDC008.004, PDC015.091, PDC020.005, and PDC020.011 are identical.
2. Mass Spectrometry of PDC008.004, batch PDC020.031: Direct Insertion into the probe was used to obtain chemical ionization and electron impact mass spectra due to the low volatility of this compound. Chemical ionization with positive ion detection gave a strong peak at 464.43 (MH+) with fragments at 377.32 and 212.17 m/z ratio. Electron impact showed the molecular ion at 463, fragments at 377,211 and the base peak at 86 m/z ratio. The calculated molecular weight of PDC008.004 is 463.63 g/mole.
3. Ultraviolet absorption spectrum of PDC008.004: This compound has a strong absorbance from 190 to 250 nm with a shoulder at 285 nm in 1-octanol. The extinction coefficient at 285 nm in 1-octanol is 8199 ± 616 M⁻¹cm⁻¹ (average of batches PDC008.034, PDC008.055, and PDC020.023).
4. Melting points of several synthetic batches of PDC008.004: Recrystallization from acetonitrile or ethanol and water yields white flakes. Individual melting points ranged from 206-207 (one batch) and 208-209°C (four batches).
5. Thin layer chromatography of PDC008.004: The retention factor for PDC008.004 is 0.2 on silica gel GF (fluorescent), 250 micrometers, using 1:9:90 ammonium hydroxide/methanol/chloroform to elute with visualization by UV or iodine.
6. High performance liquid chromatography of PDC008.004: PDC008.004 elutes at 8.4 minutes and is visualized at 285 nm. Standard conditions are gradient elution from 50% to 100% methanol (with 1% tetrahydrofuran and 1% triethylamine) in water with a linear gradient at a flow rate of 2 mL/min using a 10 cm C18 column.
7. Partition coefficient for PDC008.004: log P was determined to be 0.78 ± 0.18. Equilibrations were carried out at 23°C by dissolving PDC008.004 in 1-octanol (0.01-0.1 mM) and stirring with an equivalent volume of 0.1 M sodium phosphate at pH 7.4 for between one and 24 hours. The partition coefficient is independent of concentration or equilibration time.

### Example 3: Biochemical Pharmacology of 11-[[[2(diethylamino)ethyl] cyclohexylmethylamino]-acetyl]-5,11-dihydro-6H-pyrido [2,3-b] [1,4]-benzodiazepin-6-one (PDC008.004).

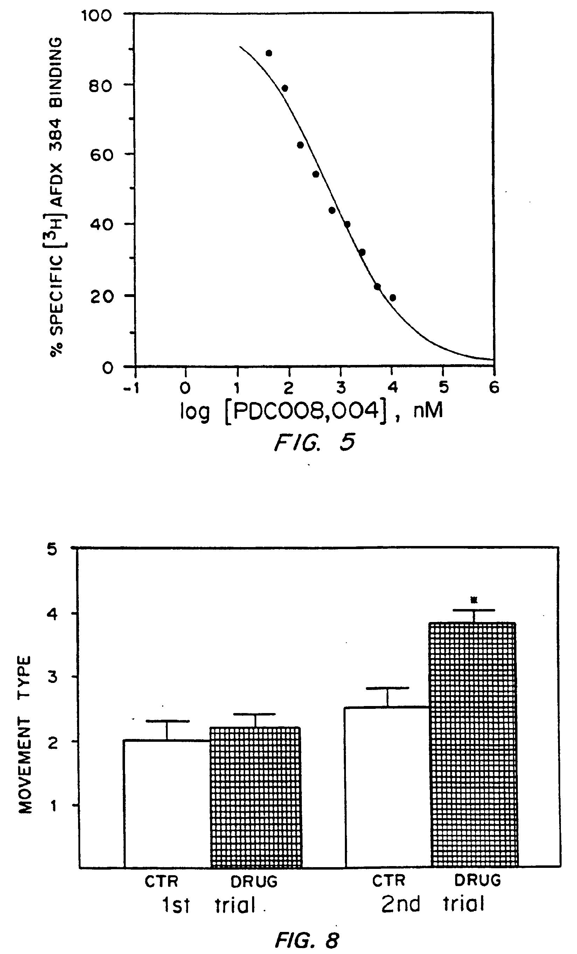

Studies examining inhibition of [³H]AF-DX-384 binding to M₂ receptors in the rat brain by PDC 008.004, yielded an Ki of 422 nm, as shown in Figure 5. These results indicate that this compound has an affinity for M₂ receptors in the nanomolar range. Thus, compounds having an affinity in the nanomolar range most likely possess therapeutic effectiveness.

High performance liquid chromatography was used to determine the concentration in the brain and mass spectrometry was used to confirm that PDC008.004 was actually present.

Brain extraction procedure: Rats were injected with between 10 and 30 mg/kg PDC008.004 by I.P. and P.O. routes of administration and sacrificed 60 minutes later. The brains were removed and extracted with 4 mL methylene chloride after homogenization in 1 mL PBS. The organic layer was evaporated under a stream of argon and the sample reconstituted in 200 microliters methanol. Samples were frozen at -20°C until use.

Using high performance liquid chromatography, the concentration in the brain is about 2 micromolar by comparison with spiked brain tissue samples of known concentration. The HPLC conditions used were identical to those indicated above.

Selected ion monitoring of the mass 464 (MH+) by chemical ionization mass spectrometry showed that PDC008.004 was indeed present in the brain tissue samples at a concentration of between 1 and 10 micromolar.

### Example 4: 11-[[[2-(diethylamino)ethyl] cyclohexylmethylamino]-acetyl]-5,11-dihydro-6H-pyr ido [2,3-b] [1,4]-benzodiazepin-6-one Specificity and Selectivity Studies: Inhibition of Radioligand Binding in Various Tissues.

Tissues were prepared and binding assays performed as described previously. Membranes were assayed in the presence of the radioligands indicated in Table 2, with varying concentrations of PDC008.004. Values (mean % specific binding) were estimated using the equation Kᵢ = IC₅₀/1 + [L]/K_{D}. Hill slopes were near 1.0, which indicated unity and that binding was occurring to a single population of sites. K; values could not be established for inhibition of radioligand binding by PDC008.004, thus, were greater than the indicated concentrations. The Kᵢ for PDC008.004 inhibition of [³H]pirenzepine binding to M₁ sites was observed to be partially due to solvent interaction, as determined by solvent controls. [³H]QNB binding was performed in the added presence of 10 µM AF-DX 384. Data analysis was performed using GraphPad (ISS Software). These data clearly indicate the specificity and selectivity for the M₂ receptor subtype.

**Table 2.**

| **Summary of PDC008.004 specificity and Selectivity Studies: Inhibition of Radioligand Binding in Various Tissues.** | | | | |
|---|---|---|---|---|
| **Binding Inhibition by PDC008.004, K**_{**i**} **(nM)** | | | | |
| **Radioligand** | **Receptor** | **Brain** | **Heart** | **Bladder** |
| [³H]AF-DX 384¹ | M₂ | 422 | 444 | no binding |
| [³H]QNB² | M₁₋₅ | ^{a,c}>10,000 | ^{a,c}>10,000 | ^{a,c}>10,000 |
| [³H]Pirenzepine³ | M₁ | ^{b}2,000 | | |
| [³H]Nicotine⁴ | Nicotine | ^{a}>10,000 | | |
| [³H]Ro 15-1788⁵ | BZD | ^{a}>100,000 | | |
| [³H]DPCPX⁶ | Adenosine₁ | ^{a}>100,000 | | |
| [³H]SCH 23390⁷ | D₁ | ^{a}>10,000 | | |
| [³H]Sulpiride⁸ | D₂ | ^{a}>10,000 | | |
| [³H]5HT⁹ | 5HT | | | |
| | reuptake | ^{a}>10,000 | | |
| [³H] | | | | |
| Norepinephrine¹⁰ | Adrenergic | | | |
| | reuptake | ^{a}>10,000 | | |

### Methods Citations:

1. Aubert, et al., Eur. J. Pharmacol., 217:173-184 (1992); Entzeroth and Mayer, Biochem. Pharmacol., 40:1674-1676 (1990); Hammer, et al., Life Sci., 38:1653-1662 (1986).
2. Dawson and Wamsley, Brain Res. Bull., 25:311-317 (1990); Dawson, et al., Neurobiol. Aging, 13:25-32 (1991); Michel, et al., Eur. J. Pharmacol., 166:459-466 (1989).
3. Dawson and Wamsley, Brain Res. Bull., 25:311-317 (1990); Dawson, et al., Neurobiol. Aging, 13:25-32 (1991).
4. Martin, et al., J. Pharmacol. Exper. Therapeu., 266:157-163 (1983); Sloan, et al., Pharmacol. Biochem. Behav., 30:255-267 (1988).
5. Yamamura, et al., (eds), In: Neurotransmitter Receptor Binding, 2nd ed. (1988); McCabe, et al., FASEB J., 4:2934-2940 (1990).
6. Lee and Reddington, Brain Res., 368:394-398 (1986).
7. Billard, et al., Life Sci., 35:1885-1893 (1984).
8. Woodruff and Freedman, Neuroscience, 6:407-410 (1981).
9. Engel, et al., Naunyn Schmiedeberg's Arch. Pharmacol., 324:116-124 (1983).
10. Lynch and Steer, J. Biological Chem., 256:3298-3303 (1981).

### Example 5: Effect of 11-[[[2-(diethylamino)ethyl]cyclohexylmethylami no]-acetyl]-5,11-dihydro-6H-pyr ido [2,3-b] [1,4]-benzodiazepin-6-one on carbachol-mediated cAMP reductions in m₂-CHO cells.

m₂-Muscarinic receptors are well known to be negatively coupled to adenylate cyclase, so that stimulation of this receptor results in an inhibition of this enzyme. The effect can be blocked with classical muscarinic antagonists such as atropine. Adenylate cyclase converts intracellular ATP to cAMP. One way of measuring muscarinic receptor-mediated inhibition of adenylate cyclase, is to measure the resulting decrease in intracellular cAMP levels. However, since those levels are initially low, it is easier to first stimulate adenylate cyclase with, e.g., forskolin, which will elevate cAMP levels, then look at subsequent decreases in cAMP levels.

The following experiment was designed to determine whether PDC008.004 behaves like a muscarinic antagonist in blocking carbachol-mediated inhibition of cAMP levels in m₂-transfected CHO cells.

### Methods

CHO cells transfected with m₂ muscarinic receptors were obtained from Dr. Mark Brann (Univ. of Vermont) and grown to 90% confluency in 24-well plates. Since intracellular cAMP is metabolized, cells were pre-treated with 1 mM IBMX (a phosphodiesterase inhibitor) in DMEM-HEPES pH 7.2. Antagonists (either AQ-RA 741 or PDC008.004) and agonists (5 µM carbachol) were then added and preincubated for 10 min, then adenylate cyclase was activated with 1 µM forskolin. After 10 min at 37°C, the reaction was stopped, and the cyclic nucleotides were extracted and assayed for cAMP by RIA.

### Results

Carbachol-stimulation of m₂-transfected CHO cells resulted in an approximately 50% inhibition of intracellular cAMP levels. Baseline cAMP values were 5.4 ± 0.1 pmol of cAMP. This inhibition was completely blocked by atropine (1 µM) and AQ-RA 741 (1 µM). Dose-response curves for AQ-RA 741 and for PDC008.004 yielded IC₅₀ values of 0.17 µM and 5.2 µM, respectively. The IC₅₀ of PDC008.004 averaged 3.7 ± 1.0 µM in three separate experiments. Cells treated with 10 µM of PDC008.004 without carbachol had cAMP levels (4.9 ± 0.5 pmoles) that were indistinguishable from untreated cells, thus demonstrating that PDC008.004 had no agonist-activity.

### Conclusions

PDC008.004 is an m₂-muscarinic antagonist that has an IC₅₀ of 3.7 ± 1.0 µM for inhibition of carbachol-mediated cAMP reductions in m₂-transfected CHO cells. In the same assay, the muscarinic antagonist AQ-RA 741 has an IC₅₀ of 0.17 µM. PDC008.004 has no detectable muscarinic agonist activity.

### Example 6: Effect of 11-[[[2-(diethylamino)ethyl]cyclohexylmethylami no]-acetyl]-5,11-dihydro-6H-pyr ido [2,3-b] [1,4]-benzodiazepin-6-one as a modulator of acetylcholine release in the rat brain.

In the brain, *in vitro* and *in vivo* studies have suggested that negative muscarinic autoreceptors controlling the release of acetylcholine (ACh) are predominantly of the pharmacological M₂ subtype (includes the molecularly defined m₂ and M₄ subtypes). Evidence is mostly derived from experiments using preferential M₂ receptor antagonists of the AF-DX series vs M₁ blockers, as described, for example, by Lapchak, et al., 1989. Binding sites for [³H]AF-DX 116 and effect of AF-DX 116 on endogenous acetylcholine ("ACh") release from rat brain slices. *Brain Res.,* 496, 285-294; and Quirion, et al., 1993. Muscarinic and nicotinic modulation of cortical acetylcholine release monitored by *in vivo* microdialysis in freely moving adult rats. *Synapse.* Hence, a muscarinic agonist inhibits ACh release while an M₂ antagonist facilitates it.

The aim of this study was to investigate the effects of PDC008.004 on *in vitro* ACh release in the rat hippocampal brain slices. Data obtained were compared to those of a prototypic M₂ blocker, AF-DX 116 (Lapchak).

### Materials and Methods

*In vitro* ACh release from adult rat hippocampal slices was investigated as described by Hanish, et al. 1993. Modulation of hippocampal acetylcholine release: a potent central action of interleukin-2. *J. Neurosci.,* 13, 3368-3374. In brief, adult rat hippocampal slices (0.4 mm) were prepared and superfused using Brandel superfusion apparatus for 1 hr in normal Kreb's (pH 7.4), followed by 25 mM K⁺ Krebs buffer (pH 7.4) with or without the PDC008.004 (10⁻⁹M to 10⁻⁴M) for an additional hour. The superfusates were then collected, extracted and then analyzed for acetylcholine using radioenzymatic method. The data were analyzed using the Spreadsheet Software Lotus 1-2-3.

### Results

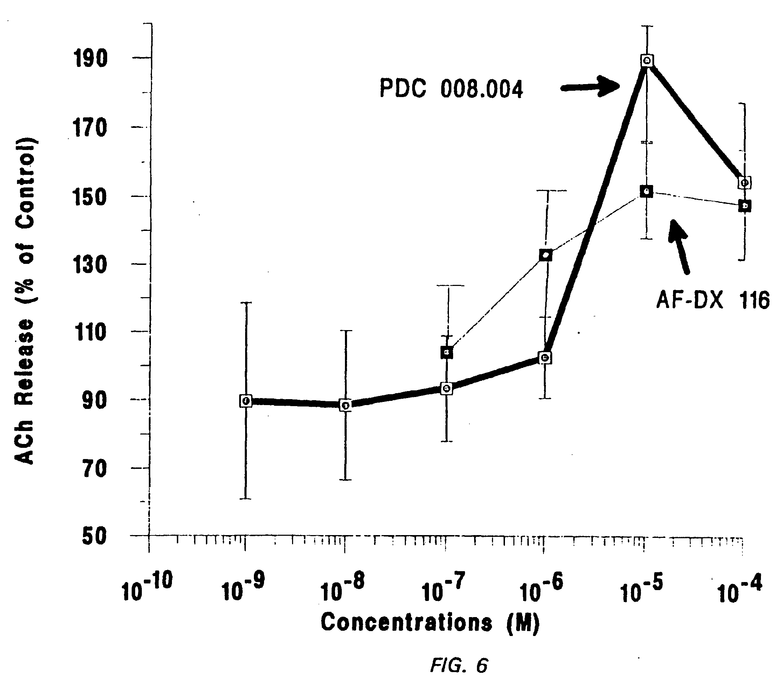

PDC008.004 significantly stimulated ACh release at µM concentrations, while having no statistically significant effects at lower concentrations, as shown in Figure 6. The maximal effect on ACh release was observed at 10⁻⁵ M; a somewhat lower potency being seen at 10⁻⁴ M. Similar results were obtained using AF-DX 116, as also shown in Figure 6; the maximal effect of PDC008.004 being slightly higher than that of AF-DX 116 (189 vs 152% over controls).

When data are expressed as a function of time over a six minute period, it can be seen that PDC008.004 transiently (first 20 minute) inhibited ACh release at nM concentrations (Table 3). This effect disappears over a full sixty minute period.

### Discussion

It is clear from these data that PDC008.004 behaves as a potent antagonist of the negative M₂ autoreceptors in the rat brain. In fact, this molecule is slightly more potent than AF-DX 116, the prototypic M₂-blocker, to facilitate ACh release. PDC008.004 is therefore one of the most potent molecules tested so far in this model. The significance of the transient inhibitory action detected at nM concentrations is unclear at the present time. It may relate to the differential affinity of PDC008.004 for m₂ vs m₄ receptors; both acting as autoreceptors but having opposite effects on ACh release.

**Table 4.**

| **Effects of PDC008.004 on K-evoked Ach release in the hippocampus, *in vitro* and as the function of time.** | | | |
|---|---|---|---|
| **TIME (min)** | **CONT PDC** | **C-SEM** | **PDC-SEM** |
| (10⁻⁹ M) | | | |
| 20 | 100 47.00631 | 27.50466 | 16.85700 |
| 40 | 100 110.9787 | 13.34888 | 32.21079 |
| 60 | 100 99.84478 | 30.99223 | 37.31391 |
| Total | 100 88.45126 | 20.60902 | 21.97463 |
| | | | |
| **TIME (min)** | **CONT PDC** | **C-SEM** | **PDC-SEM** |
| (10⁻⁸M ) | | | |
| 20 | 100 62.66493 | 36.04984 | 36.53902 |
| 40 | 100 100.9279 | 27.60613 | 41.74224 |
| 60 | 100 99.74587 | 36.99589 | 38.32475 |
| Total | 100 89.59508 | 20.43606 | 28.86637 |
| **TIME (min)** | **CONT PDC** | **C-SEM** | **PDC-SEM** |
| (10⁻⁷ M) | | | |
| 20 | 100 73.87743 | 7.306799 | 23.42092 |
| 40 | 100 116.9088 | 18.38595 | 14.61358 |
| 60 | 100 94.19564 | 14.71210 | 13.73070 |
| Total | 100 93.47309 | 9.078878 | 15.46531 |
| **TIME (min)** | **CONT PDC** | **C-SEM** | **PDC-SEM** |
| (10⁻⁶ M) | | | |
| 20 | 100 79.40970 | 24.47207 | 10.87415 |
| 40 | 100 106.8592 | 33.62842 | 10.30880 |
| 60 | 100 118.5762 | 23.76090 | 22.11482 |
| Total | 100 102.6134 | 25.23657 | 12.04337 |
| **TIME (min)** | **CONT PDC** | **C-SEM** | **PDC-SEM** |
| (10⁻⁵ M) | | | |
| 20 | 100 255.5860 | 66.74166 | 33.88827 |
| 40 | 100 282.2882 | 76.64119 | 25.43840 |
| 60 | 100 130.2478 | 35.19295 | 25.50158 |
| Total | 100 189.9553 | 30.11606 | 23.45691 |
| **TIME (min)** | **CONT PDC** | **C-SEM** | **PDC-SEM** |
| (10⁻⁴ M) | | | |
| 20 | 100 172.6805 | 37.37869 | 28.29408 |
| 40 | 100 158.9299 | 31.03878 | 26.39601 |
| 60 | 100 140.1262 | 30.62706 | 19.19002 |
| Total | 100 154.7652 | 22.99291 | 22.94442 |

### Example 7: Toxicity Studies of 11-[[[2-(diethylamino)ethyl]cyclohexylmethylami no]-acetyl]-5,11-dihydro-6H-pyr ido [2,3-b] [1,4]-benzodiazepin-6-one.

The effect of various doses of PDC008.004 on heart rate in male, Sprague-Dawley rats which received cumulative doses of PDC008.004 (0.1 to 31.1 mg/kg), where heart rate was assessed by EKG approximately 15 minutes after each dose, was determined. Control animals received an equal volume of vehicle (Emulphor:saline:DMSO, 18:72:10%) or (-)norepinephrine bitartrate (1 mg/rat).

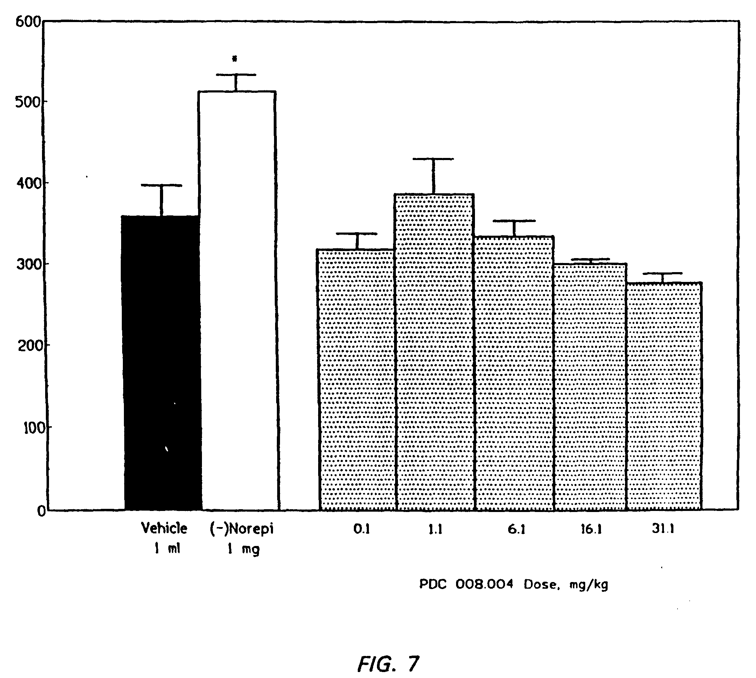

The results are shown in Figure 7. These data demonstrate that PDC008.004 did not significantly alter heart rate compared to vehicle controls. Administration of (-)norepinephrine bitartrate significantly increased heart rate 43% above vehicle control.

### Example 8: Evaluation of 11-[[[2-(diethylamino)ethyl]cyclohexylmethylami no]-acetyl]-5,11-dihydro-6H-pyr ido [2,3-b] [1,4]-benzodiazepin-6-one in Watermaze Behavioral Studies

The following procedures are standard techniques routinely used by those skilled in the art (Morris, R.G.M., 1981)

### Materials

### 1. ANIMALS

Male C57BL/6 mice, 30 g body weight. Minimum 50 animals. Obtainable from The Jackson Laboratories, Bar Harbor, Rt. 3, Maine 04609. CL-1 mice were also used, with the same results.

The animals need at least one week of acclimatization to the colony prior to experiments.
Housing: standard environment but as quiet as possible.

### 2. EQUIPMENT

### 2A. Water tank and accessories:

Circular water tank made of sturdy (10 mm thick, minimum) black (or very dark, opaque) lucite or other plastic. Diameter of the tank: 1 meter; Wall height: 509 cm. The tank must have a valve connected to a large diameter (min 1.5 cm inner) water filling/draining rubber hose (min. length 10 ft).

### 2B. Targets:

Two targets, one made of the same plastic as the water tank (the "black target"), and one made of translucent variety. Base made of heavy lucite (1 inch thick) shaped as a square with 10 inch sides. Height between the upper surface of the base and the lower surface of the target platform: 20 cm. Target platform support: 20 cm tall, circular rod (1 inch diam.). Circular target platform, 8 cm across, made of 0.5 cm thick plastic and covered with a non-skid material (surgical tape can be used) painted either black or white (depending on the target).

Target cues: White sheets, either to be suspended above the rim of the tank wall or from the rim of the tank wall, and which depict easily recognizable patterns.

A supply of powdered skim milk to cloud the water in the tank. Tank cleaning supplies (Alconox, sponges, *etc*.)

### 2B. Data collecting equipment

A standard personal computer with hard disk and 3.5 inch floppy drives, monitor and keyboard on a movable stand. The computer should be devoted to the study during its duration.

WATERMAZ©data collecting program Infallible Software, Inc., Durham, N.C.).

Statistics software.

### 3. Environment

The water tank was placed in a space with access to a sink and both cold and hot water taps. The tank was accessible from two sides. The space was provided by a quiet, isolated room with lockable door and not too bright fluorescent ceiling illumination. The room had a stable temperature of approximately 22°C.

### 4. Methods

### 4A. Regimens:

Regimen "A" consists of concurrent injections of either drug(s) or saline and swimming. Injections administered in a sequence: injection - 24h - swim - 2h - injection - 24h - swim - 2h - injection . . . etc., until a stable "time to target" plateau among the controls will be reached. If desired, this regimen can be extended for a period of additional 5 to 7 days to observe whether the data in the treated group(s) will eventually change with respect to controls.

Regimen "B" consists of saline or drug injected animals with agents administered for 7 days prior to swimming, followed by one day injection free (washout period) and training to target until a stable control plateau is reached.

Mice exhaust rather rapidly. Therefore, unless absolutely necessary, "mass trials" (*i.e.*, more than one trial a day) are not advised because physical stress factor may be inadvertently introduced. Single trial/day eliminates the problem.

Ten animals/group provides sufficiently high statistical reliability. However, training of more than 40 animals/day (*i.e.*, four groups) is impractical because of interruptions necessary for refilling the tank with fresh water, injection sequence problems, and of a vast amount of generated data that will subsequently reduce the efficiency of statistical analysis (some programs may not accept very large such numbers of data entries).
Course of the experiment and data collected:

The experiments were conducted in the following sequence: Time to submerged target (7 days) - free swim [target removed (1 day)] - time to submerged target (7 days) - free swim (1 day) . . . etc. (until plateau) - reversed target [target in new position (until plateau, typically 3 to 5 days] - visible (black target, until plateau). Maximum time to target allowed: 120 sec.
Maximum time in tank during the free swim: 60 sec.
Data: Time to target
Quadrant preference (time/quadrant)
Immobility
Number of center crossings
Movement pattern

### Results

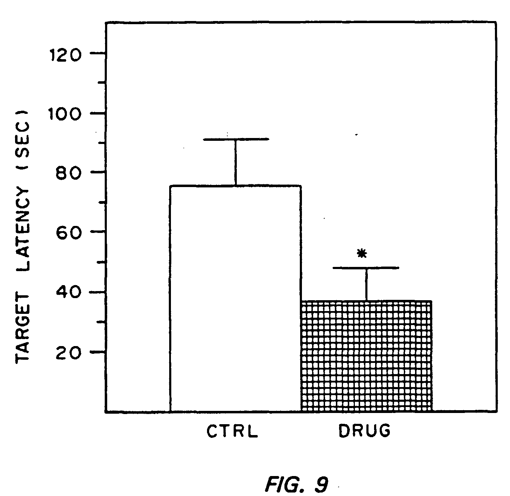

The results are shown in Figures 8 and 9. The results are for 10 control animals treated intraperitoneally with 10 mg/kg vehicle, and 10 animals treated intraperitoneally with 10 mg/kg PDC008.004. Figure 9 indicates a substantial and significant (P < 0.01) movement pattern change in the drug (11-[[[2-(diethylamino)ethyl] cyclohexylmethylamino]-acetyl]-5,11-dihydro-6H-pyrido [2,3-b][1,4]-benzodiazepin-6-one) treated mice consisting of a rapid shift from thygmotactic (wall-hugging) to center crossing and center crossing-goal oriented behavior. This behavior is consistent with increased motor activity seen after a single injection of the drug (10 mg/kg, intraperitoneally) followed by a 24 hour period prior to behavioral testing. In the control group, a slow shift from thygmotaxy to thygmotaxy/center crossing is evident, however, wall hugging is predominant. Behavior of the controls is consistent with that of saline-injected animals observed in several previous studies. Morris, R.G.M., *Learning and Motivation,* 1981.

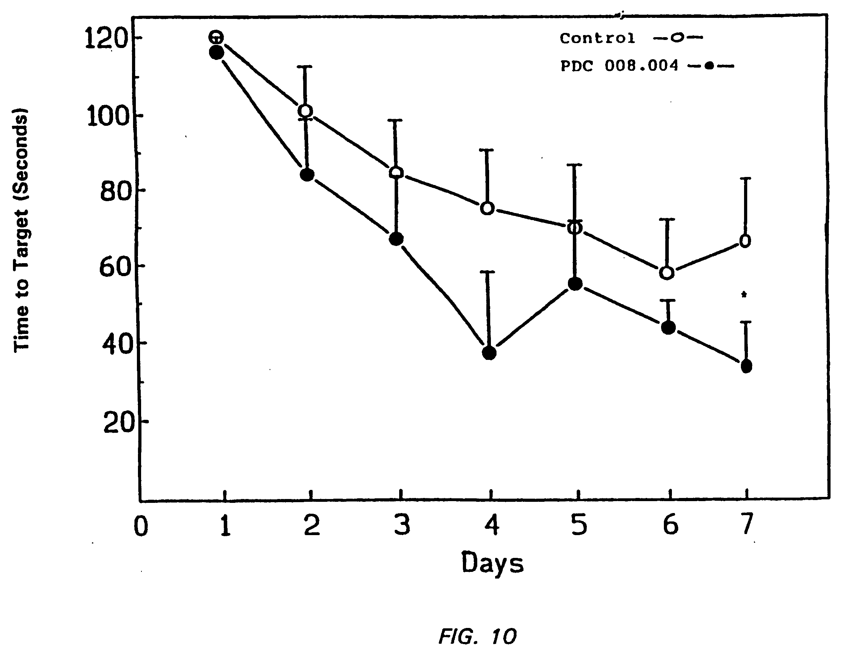

Figure 10 indicates a substantial statistically significant (P < 0.05) decrease in the time required to find a hidden target after the fourth trial using mice treated with daily injections of PDC 008.004 (10 mg/kg, intraperitoneally).

These results indicate that the observed effects are directly related to central effects of M₂ receptors by the administered agent, PDC 008.004 (10 mg/kg, intraperitoneally, daily). Moreover, the presence of dyskinesia, ataxia, and absence of flaccid paralysis observed in animals injected with the drug at 100 mg/kg intraperitoneally, supports this conclusion. The very swift appearance (maximum of 2 min.) of behavioral signs (dyskinesia, and central cholineric mediated events) demonstrates rapid penetration into the intracerebral space.

Additional studies were conducted which indicate that similar results were obtained in two different strains of mice, C57BL/6 and CL-1. These results are shown in Table 4.

**Table 4:**

| **Comparison of Two Strains of Mice** | | |
|---|---|---|
| **Mouse Strain** | **Intraperitoneal (I.P.)** | **Oral (P.O.)** |
| C57/BL/6 | 37.5 mg/kg | 50 mg/kg |
| CL-1 | 37.5 mg/kg | 50 mg/kg |

Completed preliminary results using the Morris Watermaze behavioral model demonstrated an effective dose of approximately 5 to 10 mg/kg (I.P. and P.O.). Based on these data, the TI (therapeutic index) is between 7.5 and 10 for I.P. and P.O. administration, respectively. Additional studies indicate that the effective dose of PDC008.004 may be less than 5 mg/kg, with a correspondingly better TI.

In conclusion, the results obtained during four behavioral trials demonstrate the potential therapeutic applications of the agent, PDC 008.004, and its related compounds in acute and chronic disorders in which cognitive processes are severely affected. Examples of such disorders are: stroke, global cerebral ischemia (after cardiac arrest), Alzheimer's, and related disorders of advanced age (*i.e.*, senile dementia), Parkinson's and Huntington's diseases, chronic addictive disorders (*i.e.*, alcoholism), and inflammatory disorders of the brain (*i.e.*, meningitis). Neonatal hypoxia and related cognitive deficits may also be amenable to such treatment.

Modifications and variations of the present invention will be obvious to those skilled in the art from the foregoing detailed description. Such modifications and variations are intended to come within the scope of the appended claims.

## Claims

1. A compound having the formula: 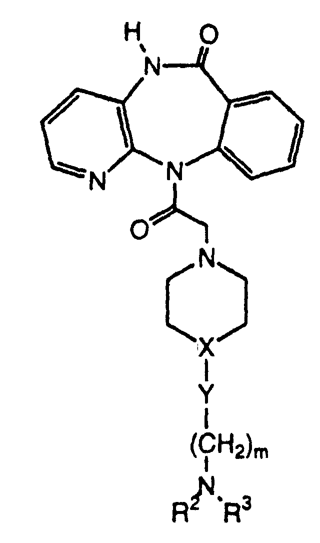
X = -CH, N;
Y = -CONR¹-, -NR¹CO-, -CH₂NR¹-, -NR¹CH₂-, -NCO(CH₂)ₐNR;
R¹ = alkyl such as methyl, isopropyl, or t-butyl; cycloalkyl such as cyclopentyl, cyclohexylmethyl, or adamantyl; arylalkyl such as benzyl, napthylmethyl, or phenylpropyl; alkyl- or arylcarbonyl, such as cyclohexane carbonyl or benzoyl; alkyl- or aryl sulfonyl, such as methane sulfonyl or napthalenosulfonyl; alkyl- or arylurea, such as cyclohexylurea or phenylurea; but are not limited to these alkyl or aryl groups, and are optionally substituted with the following groups: alkyl, aryl, alkoxy, aryloxy, amino, halogen, nitro, alkyl or aryl carbonyl, alkyl or aryl sulfonyl, alkyl or aryl sulfamido, alkyl or aryl carbonamido, alkyl or arylurea;
R², and R³ = alkyl such as methyl, isopropyl, or t-butyl; cycloalkyl such as cyclopentyl, cyclohexylmethyl, or adamantyl; arylalkyl such as benzyl, napthylmethyl, or phenylpropyl; but are not limited to these alkyl, aryl cycloalkyl, or arylalkyl groups, and are optionally substituted with the following groups: alkyl, aryl, alkoxy, aryloxy, amino, halogen, nitro, alkylthio or arylthio, alkyl or aryl carbonyl, alkyl or aryl sulfonyl, alkyl or aryl sulfamido, alkyl or aryl carbonamido, alkyl or arylurea;
wherein m and n is between 1 and 20,
wherein the compound selectively binds to the muscarinic₂ receptor, crosses the blood brain barrier, and has a therapeutic index greater than 2 in enhancing cognition.

2. The compound of claim 1 wherein the compound is C₂₇H₃₇N₅O₂.

3. The compound of claim 1 further comprising a pharmaceutically acceptable carrier for administration to a patient.

4. The compound of claim 3 wherein the pharmaceutical carrier is selected from the group consisting of carriers for intravenous administration, oral administration, administration via the respiratory tract, subcutaneous administration, intramuscular administration, rectal administration, and topical administration.

5. The compound of claim 1 selected from the group consisting of 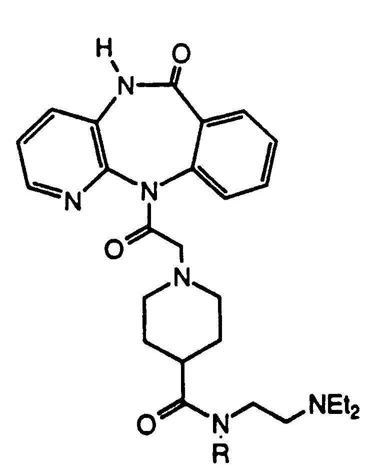 wherein 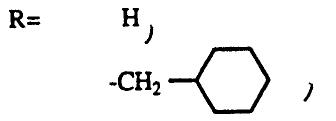 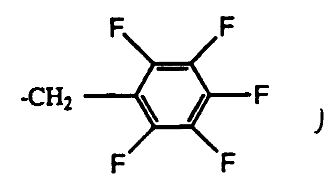
-CH₂(CH₂)₁₃CH₃ , 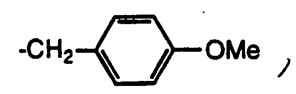
or

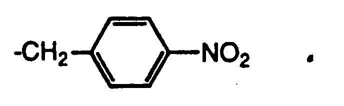

6. A method of making a pharmaceutical composition for enhancing cognition in a subject in need thereof comprising placing an effective amount of a compound according to any one of claims 1, 2 or 5 in a pharmaceutically acceptable carrier.

7. The method of claim 6, wherein the pharmaceutically acceptable carrier is selected from the group consisting of carriers for intravenous administration, oral administration, administration via the respiratory tract, subcutaneous administration, intramuscular administration, rectal administration, and topical administration.

8. A pharmaceutical composition comprising a compound according to any one of claims 1, 2 or 5 and a pharmaceutically acceptable carrier.

9. The compound according to any one of claims 1, 2 or 5 for use in medicine.

10. Use of a compound according to any one of claims 1, 2 or 5 in the manufacture of a medicament for treating a patient in need of cognitive enhancement.

11. The use according to claim 10 wherein the subject in need of cognitive enhancement suffers from a disease or disorder selected from the group consisting of Alzheimer's disease, Parkinson's disease, Huntington's disease, senile dementia, alcoholism, meningitis, neonatal hypoxia, stroke, and global cerebral ischemia.
